(19) Europäisches Patentamt
European Patent Office
Office européen des brevets

(11) Veröffentlichungsnummer: **0 204 242**
**A2**

(12) **EUROPÄISCHE PATENTANMELDUNG**

(21) Anmeldenummer: 86107120.7

(22) Anmeldetag: 26.05.86

(51) Int. Cl.⁴: C 07 D 231/16
C 07 D 401/04, A 01 N 43/56

(30) Priorität: 07.06.85 DE 3520329

(43) Veröffentlichungstag der Anmeldung:
10.12.86 Patentblatt 86/50

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Gehring, Reinhold, Dr.
Dasnöckel 49
D-5600 Wuppertal 11(DE)

(72) Erfinder: Jensen-Korte, Uta, Dr.
Gelbelstrasse 9
D-4000 Düsseldorf 1(DE)

(72) Erfinder: Schallner, Otto, Dr.
Noldeweg 22
D-4019 Monheim(DE)

(72) Erfinder: Stetter, Jörg, Dr.
Gellertweg 4
D-5600 Wuppertal 1(DE)

(72) Erfinder: Santel, Hans-Joachim, Dr.
Grünstrasse 9a
D-5090 Leverkusen 1(DE)

(72) Erfinder: Schmidt, Robert R., Dr.
Im Waldwinkel 110
D-5060 Bergisch Gladbach 2(DE)

(54) **1-Aryl-4-cyano-5-halogenpyrazole.**

(57) Die Erfindung betrifft neue 1-Aryl-4-cyano-5-halogenpyrazole der Formel (I)

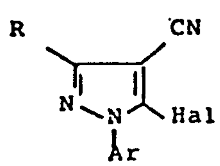

(I),

in welcher
R für Wasserstoff oder Alkyl steht,
Hal für Halogen steht und
Ar für substituiertes Phenyl oder für mehrfach substituiertes Pyridyl steht,
mehrere Verfahren zu ihrer Herstellung und ihre Verwendung als Herbizide.

EP 0 204 242 A2

0204242

BAYER AKTIENGESELLSCHAFT 5090 Leverkusen, Bayerwerk
Konzernverwaltung RP
Patentabteilung KM/Em-c
Ib

## 1-Aryl-4-cyano-5-halogenpyrazole

Die Erfindung betrifft neue 1-Aryl-4-cyano-5-halogenpyrazole, mehrere Verfahren zu ihrer Herstellung und ihre Verwendung als Herbizide.

Es ist bereits bekannt, daß 1-Aryl-4-cyanopyrazole, wie beispielsweise das 4-Cyano-5-propionamido-1-(2,4,6-trichlorphenyl)-pyrazol herbizide, insbesondere auch selektiv herbizide Eigenschaften besitzen (vgl. z.B. DE-OS 3 226 513).

Die herbizide Wirksamkeit dieser vorbekannten Verbindungen gegenüber Schadpflanzen ist jedoch ebenso wie ihre Verträglichkeit gegenüber wichtigen Kulturpflanzen nicht immer in allen Anwendungsbereichen völlig zufriedenstellend.

Weiterhin sind 4-Cyanopyrazole wie beispielsweise 1-Phenyl-4-cyano-5-brom-pyrazol und 1-(5-Nitropyrid-2-yl)-4-cyano-5-brom-pyrazol bekannt, welche als Zwischenprodukte zur Herstellung von heterocyclischen Systemen verwendet werden können (vgl. Rev. Latinoam Quim 13, 100-102 (1982). Es ist jedoch nichts über die Anwendung dieser Verbindungen in der Landwirtschaft erwähnt.

Es wurden neue 1-Aryl-4-cyano-5-halogenpyrazole der allgemeinen Formel (I),

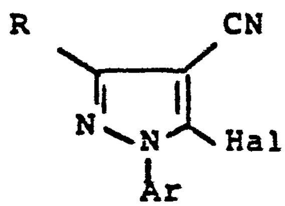

(I)

in welcher

R für Wasserstoff oder Alkyl steht

Hal für Halogen steht und

Ar für substituiertes Phenyl steht oder für mehrfach substituiertes Pyridyl steht,

gefunden.

Weiterhin wurde gefunden, daß man die neuen 1-Aryl-4-cyano-5-halogen-pyrazole der allgemeinen Formel (I),

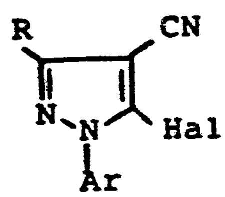

(I)

in welcher

R für Wasserstoff oder Alkyl steht,

Hal für Halogen steht und

Ar für substituiertes Phenyl steht oder für mehrfach substituiertes Pyridyl steht,

erhält, wenn man

(a-α) Pyrazolinone der Formel (II),

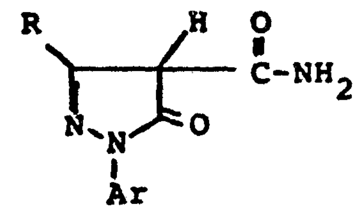

(II)

in welcher

R und Ar die oben angegebene Bedeutung haben,

oder

(a-β) 5-Hydroxypyrazol-Ammoniumsalze der Formel (III),

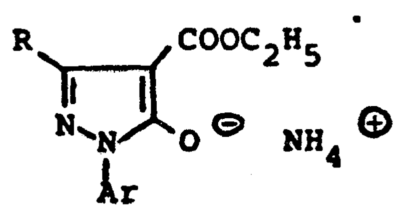

(III)

in welcher

R und Ar die oben angegebene Bedeutung haben,

mit Phosphoroxyhalogeniden der Formel (IV),

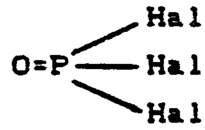

(IV)

in welcher

Hal die oben angegebene Bedeutung hat,

umsetzt,

oder wenn man

(b) 4-Hydroximinomethyl-pyrazole der Formel (V),

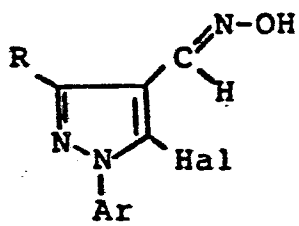

(V)

in welcher

R, Hal und Ar die oben angegebene Bedeutung haben,

mit Acetanhydrid gegebenenfalls in Gegenwart eines Verdünnungsmittels umsetzt,

oder wenn man

(c) 5-Amino-4-cyano-pyrazole der Formel (VI),

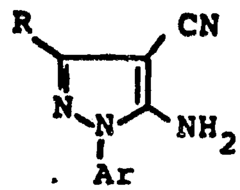

(VI)

in welcher

R und Ar die oben angegebene Bedeutung haben,

mit Natriumnitrit in Gegenwart einer Halogenwasserstoffsäure der Formel (VII),

H-Hal (VII)

in welcher

Hal die oben angegebene Bedeutung hat,

gegebenenfalls in Gegenwart eines Verdünnungsmittels umsetzt,

oder wenn man

(d) die nach Verfahren (a), (b) oder (c) erhältlichen 1-Aryl-4-cyano-5-halogenpyrazole der Formel (I),

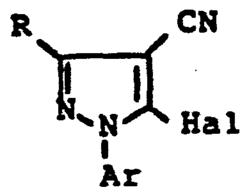

(I)

in welcher

R, Hal und Ar die oben angegebenen Bedeutungen haben,

mit Halogeniden der Formel (VIII),

$M^{\oplus}$ $(Hal^{\ominus})^1$ (VIII)

in welcher

$M^{\oplus}$ für ein Äquivalent eines Metallkations oder für ein gegebenenfalls substituiertes Ammoniumion steht und

$(Hal^{\ominus})^1$ für ein Halogenanion steht, aber verschieden von Hal ist,

gegebenenfalls in Gegenwart eines Verdünnungsmittels und gegebenenfalls in Gegenwart eines Phasentransferkatalysators umsetzt.

Auf diese Weise erhält man aus einem bestimmten Halogenderivat der 1-Aryl-4-cyano-5-halogenpyrazole der Formel (I), z.B. dem Chlorderivat, durch Umhalogenierung, z.B. mit Natriumjodid, die entsprechenden Halogenderivate der 1-Aryl-4-cyano-5-halogenpyrazole der Formel (I), in denen z.B. Chlor durch Jod ersetzt ist.

Schließlich wurde gefunden, daß die neuen 1-Aryl-4-cyano-5-halogenpyrazole der allgemeinen Formel (I) herbizide, insbesondere auch selektiv-herbizide Eigenschaften besitzen.

Überraschenderweise zeigen die erfindungsgemäßen 1-Aryl-4-cyano-5-halogenpyrazole der Formel (I) bei einer vergleichbaren allgemein herbiziden Wirksamkeit gegen schwer bekämpfbare Unkräuter eine erheblich verbesserte Kulturpflanzenselektivität im Vergleich zu den aus dem Stand der Technik bekannten 1-Aryl-4-cyanopyrazolen, wie beispielsweise das 4-Cyano-5-propionamido-1-(2,4,6-trichlorphenyl)-pyrazol, welche chemisch und wirkungsmäßig naheliegende Verbindungen sind.

In den allgemeinen Formeln steht Alkyl R für Alkyl mit 1 bis 4 Kohlenstoffatomen, insbesondere für Alkyl mit 1 bis 2 Kohlenstoffatomen, wie Methyl, Ethyl, n- und i-Propyl oder n-, i-, sec- und t-Butyl. In den allgemeinen Formeln steht R ganz besonders bevorzugt für Wasserstoff oder Methyl.

In den allgemeinen Formeln steht Hal für Fluor, Chlor, Brom oder Jod, insbesondere für Fluor, Chlor oder Brom, ganz besonders bevorzugt für Chlor oder Brom.

In den allgemeinen Formeln steht Ar für einfach oder mehrfach, gleich oder verschieden substituiertes Phenyl, oder für mehrfach, gleich oder verschieden substituiertes Pyridyl, wobei als Substituenten in Frage kommen: Alkyl mit vorzugsweise 1 bis 4, insbesondere 1 oder 2 Kohlenstoffatomen, wie Methyl, Ethyl, n- und i-Propyl, n-, i-, sec- und t-Butyl; Alkoxy mit vorzugsweise 1 bis 4, insbesondere 1 oder 2 Kohlenstoffatomen wie Methoxy, Ethoxy, n- und i-Propyloxy, n-, i-, sec- und t-Butyloxy; Alkoxycarbonyl mit vorzugsweise 2 bis 4, insbesondere 2 oder 3 Kohlenstoffatomen, wie Methoxycarbonyl, Ethoxycarbonyl oder Propyloxycarbonyl; Halogenalkyl mit vorzugsweise 1 bis 4, insbesondere 1 bis 2 Kohlenstoffatomen und vorzugsweise 1 bis 9, insbesondere 1 bis 5 und ganz besonders bevorzugt 1 bis 3 Halogenatomen, wobei die Halogenatome gleich oder verschieden sind und als Halogenatome, Fluor, Chlor, Brom oder Jod, insbesondere Fluor, Chlor oder Brom, ganz besonders Fluor oder Chlor stehen; Halogenalkoxy mit vorzugsweise 1 bis 4, insbesondere 1 oder 2 Kohlenstoffatomen und vorzugsweise 1 bis 9, insbesondere 1 bis 5, ganz besonders 1 bis 3 Halogenatomen, wobei die Halogenatome für Fluor, Chlor, Brom oder Jod, insbesondere für Fluor, Chlor oder Brom und ganz besonders für Fluor oder Chlor stehen; Halogen, vorzugsweise Fluor, Chlor, Brom oder Jod, insbesondere Fluor, Chlor, Brom, ganz besonders Chlor oder Brom, Cyano oder Nitro.

In dem Fall, daß Ar für durch den Rest $-S(O)_n-R^1$ substituiertes Phenyl steht, steht $R^1$ für Alkyl mit 1 bis 4, insbesondere 1 oder 2 Kohlenstoffatomen, wie Methyl, Ethyl, n- und i-Propyl, n-, i-, sec- und t-Butyl; für

Alkylamino mit 1 bis 4, insbesondere mit 1 oder 2 Kohlenstoffatomen, wie Methylamino, Ethylamino, Propylamino oder Butylamino; für Dialkylamino mit 1 bis 4, insbesondere 1 oder 2 Kohlenstoffatomen, in den einzelnen Alkylteilen, wie Dimethylamino, Diethylamino, Dipropylamino oder Dibutylamino; für Halogenalkyl mit 1 bis 4, insbesondere 1 oder 2 Kohlenstoffatomen und 1 bis 9, insbesondere 1 bis 5, ganz besonders 1 bis 3 Halogenatomen, wobei die Halogenatome gleich oder verschieden sind, und als Halogenatome Fluor, Chlor, Brom oder Jod, besonders Fluor, Chlor oder Brom, ganz besonders Fluor oder Chlor stehen, wie Trifluormethyl; n steht für 0, 1 oder 2.

Die erfindungsgemäßen 1-Aryl-4-cyano-5-halogenpyrazole sind durch die Formel (I) allgemein definiert.

Bevorzugt sind Verbindungen der Formel (I), bei welchen

R für Wasserstoff oder für geradkettiges oder verzweigtes Alkyl mit 1 bis 4 Kohlenstoffatomen steht,

Hal für Halogen steht und

Ar für einfach oder mehrfach, gleich oder verschieden substituiertes Phenyl oder für jeweils mehrfach gleich oder verschieden substituiertes 2-Pyridyl, 3-Pyridyl oder 4-Pyridyl steht, wobei als Substituenten jeweils in Frage kommen:

Cyano, Nitro, Halogen, jeweils geradkettiges oder verzweigtes Alkyl, Alkoxy oder Alkoxycarbonyl mit jeweils bis zu 4 Kohlenstoffatomen, jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder der Rest $-S(O)_n-R^1$, wobei

$R^1$ für Amino, für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

n für eine Zahl 0, 1 oder 2 steht.

Besonders bevorzugt sind 1-Aryl-4-cyano-5-halogenpyrazole der allgemeinen Formel (I), bei welchen

R für Wasserstoff, Methyl oder Ethyl steht,

Hal für Fluor, Chlor, Brom oder Iod steht und

Ar für ein- bis fünffach, gleich oder verschieden substituiertes Phenyl oder für jeweils zwei- bis vierfach, gleich oder verschieden substituiertes 2-Pyridyl, 3-Pyridyl oder 4-Pyridyl steht, wobei als Substituenten jeweils in Frage kommen:

Cyano, Nitro, Fluor, Chlor, Brom, Iod, Methyl, Ethyl, n- und i-Propyl, n-, i-, s- und t-Butyl, für Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder der Rest $-S(O)_n-R^1$,

wobei

$R^1$ für Amino, Methylamino, Ethylamino, Dimethylamino, Diethylamino, Fluordichlormethyl, Difluormethyl, Tetrafluorethyl, Trichlorethyl, Trifluormethyl, Methyl oder Ethyl steht

und

n für eine Zahl 0, 1 oder 2 steht.

Im einzelnen seien außer den bei den Herstellungsbeispielen genannten Verbindungen die folgenden 1-Aryl-4-cyano-5-halogenpyrazole der allgemeinen Formel (I) genannt:

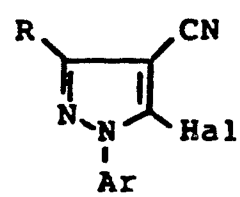

(I)

**Tabelle 1**

| R | Hal | Ar | R | Hal | Ar |
|---|---|---|---|---|---|
| H | Cl | 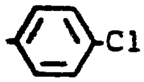 | H | Cl | 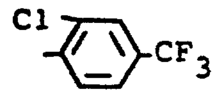 |
| H | Cl | 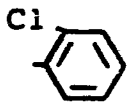 | H | Cl | 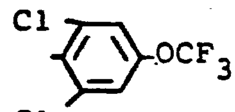 |
| H | Cl | 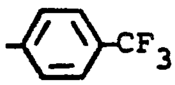 | H | Cl | 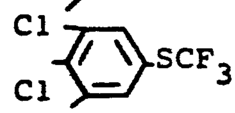 |
| H | Cl | 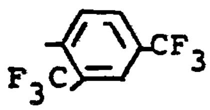 | H | Cl | 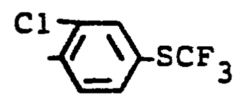 |
| H | Cl | 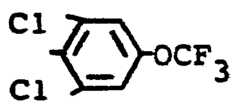 | H | Br | 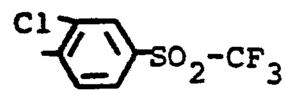 |
| H | Cl | 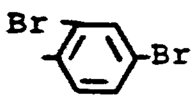 | H | Br | 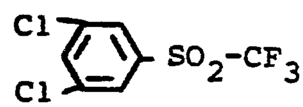 |
| H | Cl | 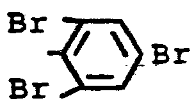 | H | Br | 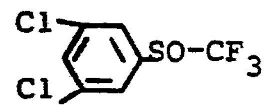 |
| H | Cl | 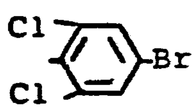 | H | Cl | 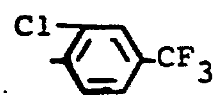 |
| H | Cl | 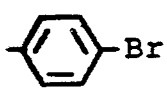 | H | Cl | 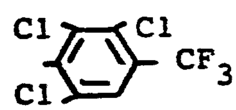 |
| H | Cl | 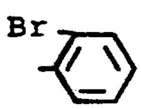 | H | Cl | 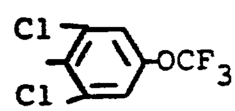 |
| H | Br | 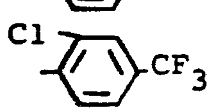 | H | Cl | 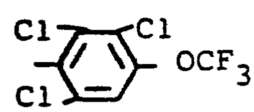 |
| H | Br | 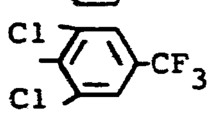 | H | Cl | 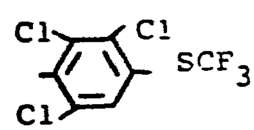 |

Tabelle 1 (Fortsetzung)

| R | Hal | Ar | R | Hal | Ar |
|---|---|---|---|---|---|
| H | Br | 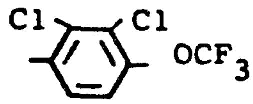 | H | Cl | 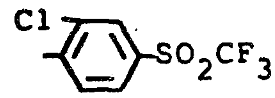 |
| H | Br | 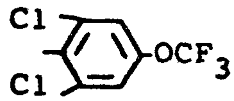 | H | Cl | 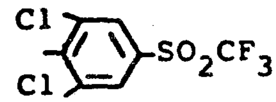 |
| H | Br | 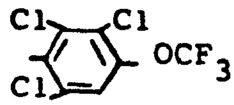 | H | Cl | 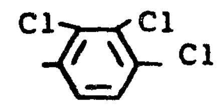 |
| H | Br | 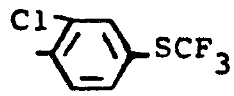 | H | Br | 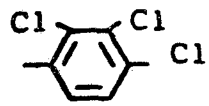 |
| H | Br | 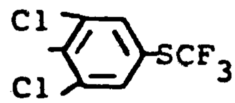 | H | Br | 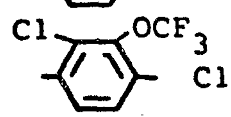 |
| H | Br | 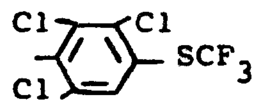 | H | Cl | 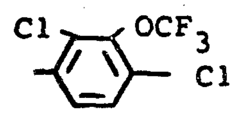 |
| H | Cl | 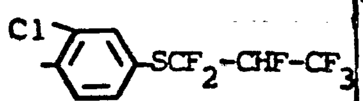 | $CH_3$ | Cl | 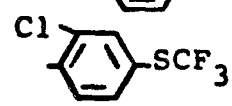 |
| H | Br | 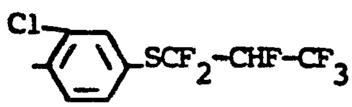 | $CH_3$ | Cl | 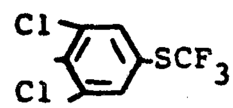 |
| $CH_3$ | Cl | 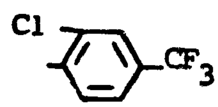 | $CH_3$ | Cl | 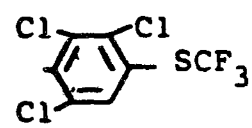 |
| $CH_3$ | Cl | 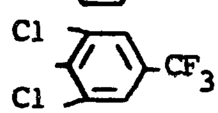 | $CH_3$ | Br | 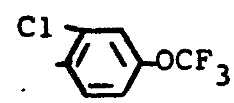 |
| $CH_3$ | Br | 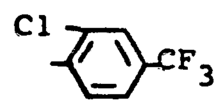 | $CH_3$ | Br | 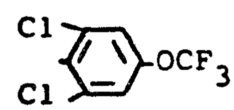 |
| $CH_3$ | Br | 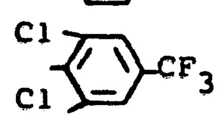 | H | Cl | 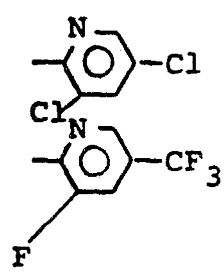 |
| | | | H | Cl | |

Tabelle 1 (Fortsetzung)

| R | Hal | Ar | R | Hal | Ar |
|---|---|---|---|---|---|
| H | Cl | 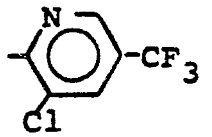 | $CH_3$ | Cl | 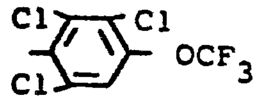 |
| H | Br | 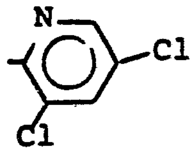 | $CH_3$ | Br | 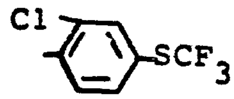 |
| H | Br | 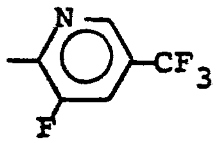 | $CH_3$ | Br | 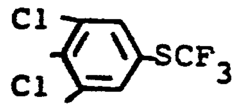 |
| $CH_3$ | Cl | 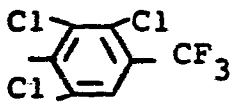 | $CH_3$ | Br | 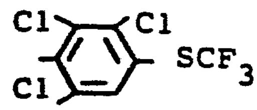 |
| $CH_3$ | Br | 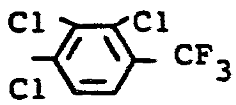 | $CH_3$ | Cl | 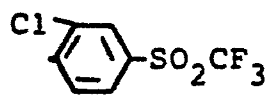 |
| $CH_3$ | Cl | 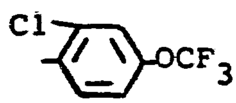 | $CH_3$ | Br | 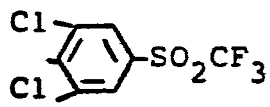 |
| $CH_3$ | Cl | 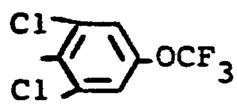 | $CH_3$ | Cl | 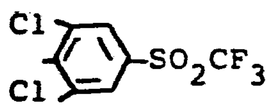 |
| | | | $CH_3$ | Br | 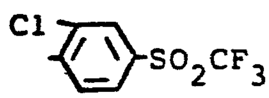 |

Verwendet man beispielsweise 1-(2,4-Dichlorphenyl)-$\Delta^2$-(1H)-pyrazolin-5-on-4-carboxamid und Phosphoroxybromid als Ausgangsstoffe, so läßt sich der Reaktionsablauf des erfindungsgemäßen Verfahrens (a-α) durch das folgende Formelschema darstellen:

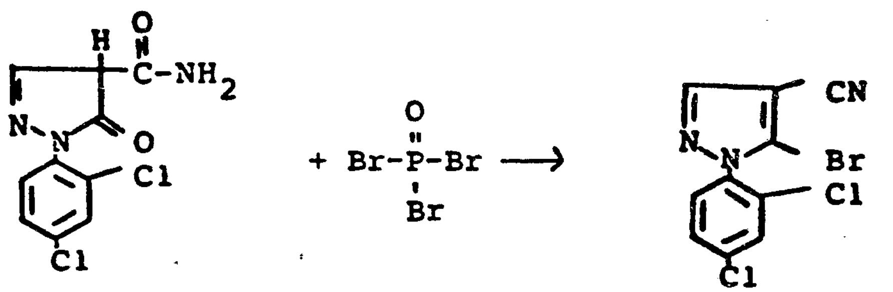

Verwendet man beispielsweise 1-(2,4-Dichlorphenyl)-4-ethoxycarbonyl-5-hydroxypyrazol- Ammoniumsalz und Phosphoroxychlorid als Ausgangsstoffe, so läßt sich der Reaktionsablauf des erfindungsgemäßen Verfahrens (a-β) durch das folgende Formelschema darstellen:

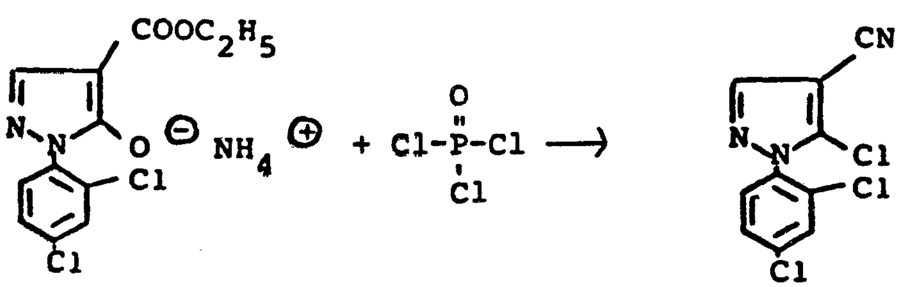

Verwendet man beispielsweise 5-Chlor-1-(2,4,6-trichlorphenyl)-4-hydroximinomethyl-pyrazol und Acetanhydrid als Ausgangsstoffe, so läßt sich der Reaktionsablauf des erfindungsgemäßen Verfahrens (b) durch das folgende Formelschema darstellen:

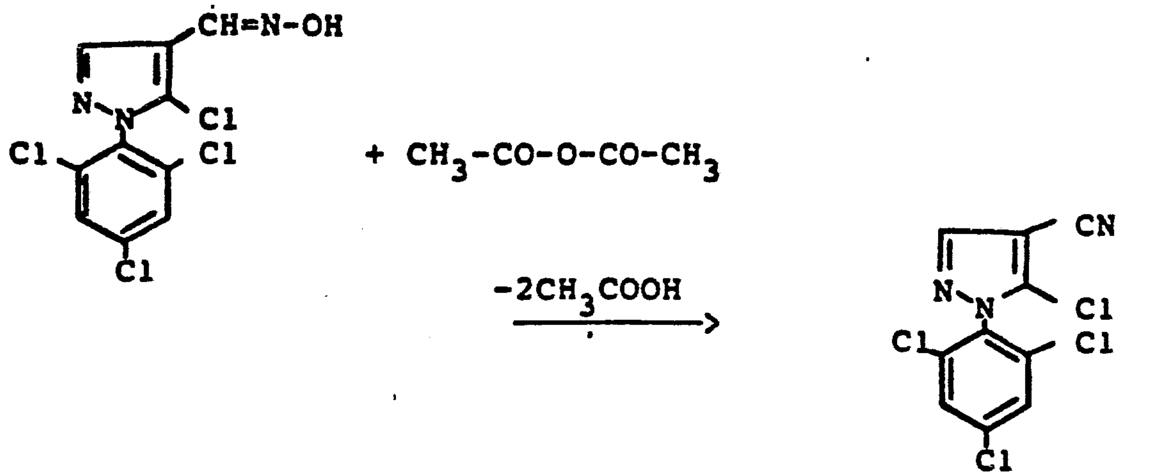


Verwendet man beispielsweise 5-Amino-4-cyano-1-(4-trifluormethylphenyl)-pyrazol und Natriumnitrit in Gegenwart von Bromwasserstoffsäure als Ausgangsstoffe, so läßt sich der Reaktionsablauf des erfindungsgemäßen Verfahrens (c) durch das folgende Formelschema darstellen:

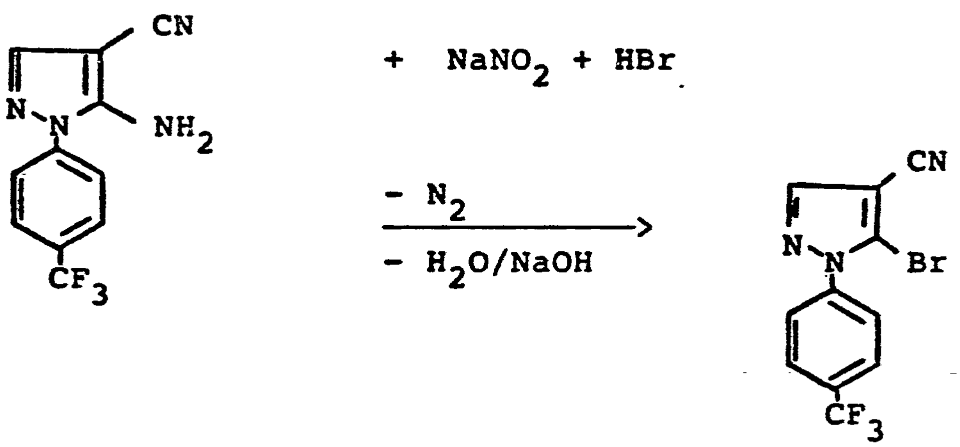

Verwendet man beispielsweise 5-Chlor-4-cyano-1-(2-chlor-4-trifluormethylphenyl)-pyrazol und Natriumfluorid als Ausgangsstoffe, so läßt sich der Reaktionsablauf des erfindungsgemäßen Verfahrens (d) durch das folgende Formelschema darstellen:

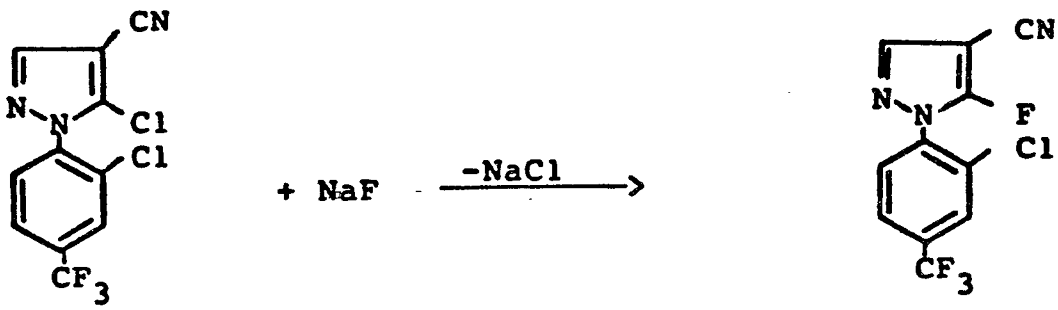


Die zur Durchführung des erfindungsgemäßen Verfahrens (a-α) als Ausgangsstoffe benötigten Pyrazolinone sind durch die Formel (II) allgemein definiert. In dieser Formel (II) stehen R und Ar vorzugsweise für diejenigen Reste, die bereits im Zusammenhang mit der Beschreibung der erfindungsgemäßen Stoffe der Formel (I) als bevorzugt für diesen Substituenten genannt wurden.

Die Pyrazolinone der Formel (II) sind noch nicht bekannt. Man erhält sie, wenn man 4-Ethoxycarbonyl-pyrazolin-5-one der Formel (IX),

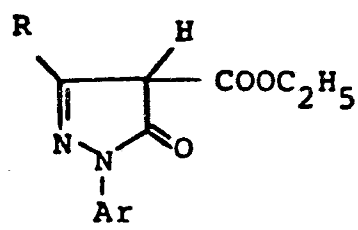

(IX)

in welcher

R für Wasserstoff oder Alkyl steht und

Ar für substituiertes Phenyl steht, oder für mehrfach substituiertes Pyridyl steht,

mit Ammoniak in Gegenwart eines Verdünnungsmittels wie beispielsweise Ethanol bei Temperaturen zwischen 80°C und 200°C umsetzt,

oder wenn man

Pyrazolin-5-one der Formel (X),

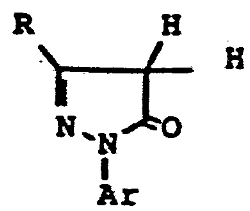

(X)

in welcher

R für Wasserstoff oder Alkyl steht

Ar für substituiertes Phenyl steht, oder für mehrfach substituiertes Pyridyl steht,

mit Harnstoff gegebenenfalls in Gegenwart eines Verdünnungsmittels wie beispielsweise Ethanol bei Temperaturen zwischen 100°C und 250°C umsetzt.

Die zur Durchführung des erfindungsgemäßen Verfahrens (a-β) als Ausgangsstoffe benötigten 5-Hydroxypyrazol-Ammoniumsalze sind durch die Formel (III) allgemein definiert. In dieser Formel (III) stehen R und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Hydroxypyrazol-Ammoniumsalze der Formel (III) sind ebenfalls noch nicht bekannt. Man erhält sie, wenn man 4-Ethoxycarbonyl-pyrazolin-5-one der Formel (IX),

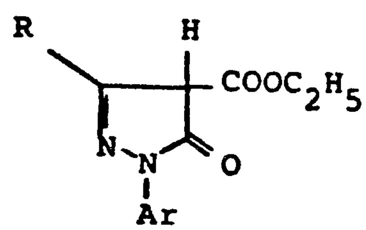

(IX)

in welcher

R und Ar die oben angegebene Bedeutung haben,

welche im Gleichgewicht mit der tautomeren Form der Formel (IX a),

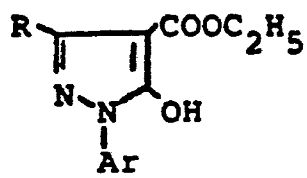

(IX a)

stehen, in welcher

R und Ar die oben angegebene Bedeutung haben,

mit wässrigem Ammoniak bei Temperaturen zwischen 20°C und 100°C umsetzt.

Die zur Durchführung des erfindungsgemäßen Verfahrens (a) weiterhin als Ausgangsstoffe benötigten Phosphoroxyhalogenide sind durch die Formel (IV) allgemein definiert. In dieser Formel (IV) steht Hal vorzugsweise für diejenigen Reste, die bereits im Zusammenhang mit der Beschreibung der erfindungsgemäßen Stoffe der Formel (I) als bevorzugt für diesen Substituenten genannt wurden.

Die Phosphoroxyhalogenide der Formel (IV) sind allgemein bekannte Verbindungen der organischen Chemie.

Die 4-Ethoxycarbonyl-pyrazolin-5-one der allgemeinen Formel (IX) sind bekannt (vgl. US-PS 4 260 751; Tetrahydron 33, 2829-2836 (1977)) oder lassen sich nach prinzipiell bekannten Verfahren in einfacher analoger Weise erhalten.

Die Pyrazolin-5-one der Formel (X) sind ebenfalls bekannt (vgl. z.B. Czech. Pat. 154 959 vom 15.09.1974, J. Ind. Chem. Soc. 52, 168 (1975) oder FR-Pat. 1 516 959 vom 15.03.1968) oder lassen sich nach bekannten Verfahren in einfacher Weise erhalten.

Die zur Durchführung des erfindungsgemäßen Verfahrens (b) als Ausgangsstoffe benötigten 4-Hydroximinomethyl-pyrazole sind durch die Formel (V) allgemein definiert. In dieser Formel (V) stehen R, Ar und Hal vorzugsweise für diejenigen Reste, die bereits im Zusammenhang mit der Beschreibung der erfindungsgemäßen Stoffe der Formel (I) als bevorzugt für diese Substituenten genannt wurden.

Die 4-Hydroximinomethyl-pyrazole der Formel (V) sind noch nicht bekannt. Man erhält sie, wenn man Pyrazolin-5-one der Formel (X),

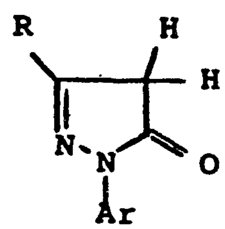

(X)

in welcher

R und Ar die oben angegebene Bedeutung haben,

oder wenn man 5-Halogenpyrazole der Formel (XI),

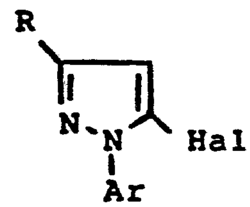

(XI)

in welcher

R und Ar die oben angegebene Bedeutung haben und

Hal für Halogen steht,

nach prinzipiell bekannten Verfahren ("Vilsmeier-Formylierung") mit Phosphoroxyhalogenid der Formel (IV),

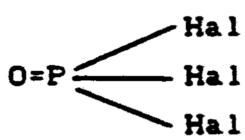

(IV)

in welcher

Hal die oben angegebene Bedeutung hat,

und einem geeigneten Formamid der Formel (XII),

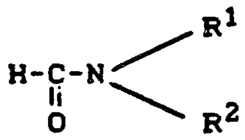

(XII)

in welcher

$R^1$ für Alkyl, insbesondere für Methyl oder Ethyl steht und

$R^2$ für Alkyl oder Aryl, insbesondere für Methyl, Ethyl oder Phenyl steht,

bei Temperaturen zwischen 50°C und 150°C umsetzt und die so erhältlichen 4-Formyl-5-halogen-pyrazole der Formel (XIII),

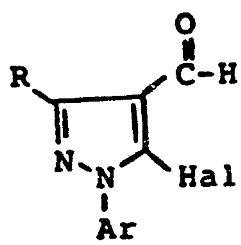

(XIII)

in welcher

R, Ar und Hal die oben angegebene Bedeutung haben,

mit Hydroxylamin gegebenenfalls in Gegenwart eines Verdünnungsmittels, wie beispielsweise Ethanol, bei Temperaturen zwischen 20°C und 150°C umsetzt.

Die 5-Halogenpyrazole der Formel (XI) erhält man wenn man Pyrazolin-5-one der Formel (X),

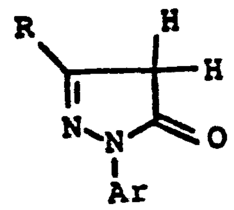

(X)

in welcher

R und Ar die oben angegebene Bedeutung haben,

mit Phosphoroxyhalogenid der Formel (IV),

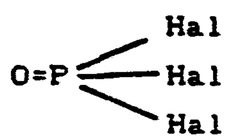

(IV)

in welcher

Hal die oben angegebene Bedeutung hat,

bei Temperaturen zwischen 100°C und 250°C umsetzt.

Die zur Durchführung des erfindungsgemäßen Verfahrens (c) als Ausgangsstoffe benötigten 5-Amino-4-cyano-pyrazole sind durch die Formel (VI) allgemein definiert.

In dieser Formel (VI) stehen R und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-4-cyano-pyrazole der Formel (VI) sind teilweise bekannt (vgl. z.B. EP 34 945; DE-OS 3 226 496; DE-OS 3 129 429 oder die noch nicht veröffentlichte eigene vorgängige Patentanmeldung DE-P 3 337 543 vom 15.10.1983) oder lassen sich nach bekannten Verfahren in analoger Weise erhalten, indem man beispielsweise Ethoxymethylenmalodinitril der Formel (XIV),

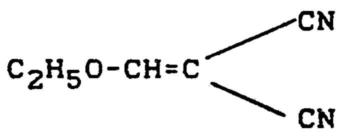

(XIV)

mit Arylhydrazinen der Formel (XV),

$$Ar-NH-NH_2 \qquad (XV)$$

in welcher

Ar die oben angegebene Bedeutung hat,

entweder zunächst in einer ersten Stufe, gegebenenfalls in Gegenwart eines Verdünnungsmittels wie beispielsweise Eisessig oder Ethanol, und gegebenenfalls in Gegenwart eines Reaktionshilfsmittels, wie beispielsweise Natriumacetat, bei Temperaturen zwischen -20° C und +20° C umsetzt zu den Arylhydrazin-Derivaten der Formel (XVI),

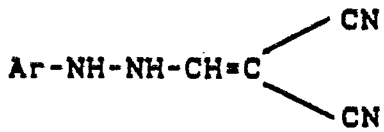 (XVI)

in welcher

Ar die oben angegebene Bedeutung hat,

und diese in einer 2. Stufe, gegebenenfalls in Gegenwart eines Verdünnungsmittels, wie beispielsweise Ethylenglykolmonoethylether bei Temperaturen zwischen +50° C und +150° C cyclisiert, oder direkt in einem Reaktionsschritt ohne Isolierung der Zwischenstufe der Formel (XVI), gegebenenfalls in Gegenwart eines Verdünnungsmittels, wie beispielsweise Ethylenglykolmonoethylether oder Ethanol, bei Temperaturen zwischen +50° C und +150° C cyclisiert.

Das Ethoxymethylenmalondinitril der Formel (XIV) und die Arylhydrazine der Formel (XV) sind bekannt, bzw. lassen sich nach bekannten Verfahren in einfacher analoger Weise herstellen (vgl. z.B. Houben-Weyl "Methoden der organischen Chemie" Band X/2, S. 203, Thieme Verlag Stuttgart, 1967), US-PS 4 127 575; US-PS 3 609 158; DE-OS 2 558 399 oder J.chem.Soc. C 167-174 (1971)).

Die zur Durchführung des erfindungsgemäßen Verfahrens (c) weiterhin als Ausgangsstoffe benötigten Halogenwasserstoffsäuren sind durch die Formel (VII) allgemein

definiert. In dieser Formel (VII) steht Hal vorzugsweise für diejenigen Reste, die bereits im Zusammenhang mit der Beschreibung der erfindungsgemäßen Stoffe der Formel (I) als bevorzugt für diesen Substituenten genannt wurden.

Die Halogenwasserstoffsäuren der Formel (VII) sind allgemein bekannte Verbindungen.

Die zur Durchführung des erfindungsgemäßen Verfahrens (d) als Ausgangsstoffe benötigten 1-Aryl-4-cyano-5-halogen-pyrazole sind durch die Formel (I) allgemein definiert.

Die 1-Aryl-4-cyano-5-halogenpyrazole der Formel (I) sind erfindungsgemäße Verbindungen und erhältlich mit Hilfe der erfindungsgemäßen Verfahren (a), (b) oder (c).

Die zur Durchführung des erfindungsgemäßen Verfahrens (d) weiterhin als Ausgangsstoffe benötigten Halogenide sind durch die Formel (VIII) allgemein definiert. In dieser Formel (VIII) steht $M^+$ vorzugsweise für ein Natrium- oder Kaliumkation oder für ein Ammonium- oder Tetraalkylammoniumion, wobei als Alkylreste solche mit 1 bis 12 Kohlenstoffatomen in Frage kommen. $(Hal^-)^1$ steht vorzugsweise für Fluor, Chlor, Brom oder Iod.

Die Halogenide der Formel (VIII) sind ebenfalls allgemein bekannte Verbindungen der organischen Chemie.

Die Verfahren (a-α) und (a-β) werden im allgemeinen ohne Zusatz eines Verdünnungsmittels durchgeführt. In der Regel dient ein entsprechender Überschuß an Phosphoroxyhalogenid als Lösungsmittel.

Die Reaktionstemperaturen können bei der Durchführung der erfindungsgemäßen Verfahren (a-α) und (a-β) in einem größeren Bereich variiert werden. Im allgemeinen arbeitet man zwischen +50° C und +200° C, vorzugsweise zwischen +100° C und +180° C.

Zur Durchführung der erfindungsgemäßen Verfahren (a-α) und (a-β) setzt man pro Mol an Pyrazolinon der Formel (II) bzw. 5-Hydroxypyrazol-Ammoniumsalz der Formel (III) im allgemeinen 1,0 bis 20,0 Mol, insbesondere 1,0 bis 10,0 Mol an Phosphoroxyhalogenid der Formel (IV) ein.

Liegt die Siedetemperatur des verwendeten Phosphoroxyhalogenids unterhalb der erforderlichen Reaktionstemperatur, so kann es gegebenenfalls erforderlich sein, unter Druck zu arbeiten. Man schließt in diesem Fall das Reaktionsgemisch im Bombenrohr oder Autoklaven ein und erwärmt für mehrere Stunden auf die erforderliche Reaktionstemperatur. Zur Aufarbeitung wird die Reaktionsmischung mit wässriger Carbonatlösung neutralisiert und abgesaugt. Aus dem Rückstand läßt sich das 1-Aryl-4-cyano-5-halogenpyrazol der Formel (I) mit einem geeigneten organischen Lösungsmittel extrahieren.

Zur Durchführung des erfindungsgemäßen Verfahrens (b) kommen als Verdünnungsmittel inerte organische Lösungsmittel in Frage, welche weiter unten näher definiert werden. Vorzugsweise verwendet man jedoch als Lösungsmittel einen entsprechenden Überschuß des gleichzeitig als Reagenz verwendeten Acetanhydrids.

Die Reaktionstemperaturen können bei der Durchführung des erfindungsgemäßen Verfahrens (b) in einem größeren Bereich variiert werden. Im allgemeinen arbeitet man zwischen 50°C und 180°C, vorzugsweise zwischen 80°C und 150°C.

Zur Durchführung des erfindungsgemäßen Verfahrens (b) setzt man pro Mol an 4-Hydroximinomethyl-pyrazol der Formel (V) im allgemeinen 1,0 bis 20,0 Mol, insbesondere 1,0 bis 10,0 Mol an Acetanhydrid ein. Die Reaktionsdurchführung, Aufarbeitung und Isolierung der Reaktionsprodukte der Formel (I) erfolgt in Analogie zu bekannten Verfahren (vgl. z.B. Zh. org. Khim. 9, 2416-2421 (1973)).

Als Verdünnungsmittel zur Durchführung des erfindungsgemäßen Verfahrens (c) kommen alle üblicherweise für derartige Diazotierungsreaktionen verwendbaren Lösungsmittel wie beispielsweise Essigsäure in Frage. Vorzugsweise verwendet man einen Überschuß an der als Reaktionspartner eingesetzten Halogenwasserstoffsäure der Formel (VII) gleichzeitig als Verdünnungsmittel.

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und +120°C, vorzugsweise bei Temperaturen zwischen -10°C und +80°C.

Zur Durchführung des erfindungsgemäßen Verfahrens (c) setzt man pro Mol an 5-Amino-4-cyanopyrazol der Formel (VI) im allgemeinen 1,0 bis 10,0 Mol an Natriumnitrit und 1,0 bis 100,0 Mol an Halogenwasserstoffsäure der Formel (VII) ein. Die Reaktionsführung, Aufarbeitung und Isolierung der Reaktionsprodukte erfolgt in Analogie zu bekannten Verfahren (vgl. z.B. "Organikum" 15. Auflage; VEB Deutscher Verlag der Wissenschaften, Berlin 1981, S. 652 ff.).

Als Verdünnungsmittel zur Durchführung des erfindungsgemäßen Verfahrens (d) kommen inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone , wie Aceton oder Butanon, Nitrile, wie Acetonitril oder Propionitril, Amide, wie Dimethylformamid, Dimethylacetamid, N-Methylformamid, N-Methylpyrrolidon oder Hexamethylphosphor-

säuretriamid, Ester, wie Essigsäureethylester, Sulfoxide oder Sulfone, wie Dimethylsulfoxid oder Sulfolan, oder Alkohole, wie Methanol, Ethanol oder Propanol.

Als Katalysatoren zur Durchführung des erfindungsgemäßen Verfahrens (d) kommen quartäre Ammonium- oder Phosphoniumsalze oder cyclische Polyether in Frage.

Als Beispiele für solche Katalysatoren seien genannt: Tetrabutylammoniumiodid, Tetrabutylammoniumbromid, Tributylmethylphosphoniumbromid, Trimethyl-$C_{13}/C_{15}$-alkylammoniumchlorid, Dibenzyl-dimethyl-ammonium-methylsulfat, Dimethyl-$C_{12}/C_{14}$-alkyl-benzylammoniumchlorid, Tetrabutylammoniumhydroxid, 18-Krone-6, Triethylbenzylammoniumchlorid, Trimethylbenzylammoniumchlorid.

Die Reaktionstemperatur kann bei der Durchführung des erfindungsgemäßen Verfahrens (d) innerhalb eines größeren Bereichs variiert werden. Sie liegt im allgemeinen zwischen 0°C und +120°C, vorzugsweise zwischen +20°C und +80°C.

Zur Durchführung des erfindungsgemäßen Verfahrens (d) setzt man pro Mol an Ausgangsverbindung der Formel (Ia) im allgemeinen 1,0 bis 50 Mol, vorzugsweise 1,0 bis 20 Mol an Halogenid der Formel (VIII) und 0,01 bis 1,0 Mol an Phasentransferkatalysator ein. Die Reaktionsdurchführung, Aufarbeitung und Isolierung der Reaktionsprodukte erfolgt in Analogie zu bekannten Verfahren (vgl. z.B. "Organikum", 15. Auflage, VEB Deutscher Verlag der Wissenschaften, Berlin 1981, S. 421 ff.).

Die erfindungsgemäßen Verbindungen der Formel (I) können neben ihrer Verwendung als herbizide Wirkstoffe auch als Zwischenprodukte zur Synthese weiterer Wirkstoffe verwendet werden. Sie lassen sich beispielsweise mit Aminen umsetzen zu den entsprechenden ebenfalls herbizid wirksamen 5-Amino-1-aryl-4-cyanopyrazolen, welche Gegenstand der EP-OS 34 945, der DE-OS 3 226 496, der DE-OS 3 129 429, sowie der eigenen vorgängigen Patentanmeldung DE-P 3 337 543 vom 15.10.1983 sind.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind.

Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

0204242

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Wirkstoffe der Formel (I) neben einer besonders guten allgemein-herbiziden Wirksamkeit auch eine deutlich verbesserte Pflanzenverträglichkeit in wichtigen Kulturen und können als selektive Unkrautbekämpfungsmittel sowohl in dikotylen Kulturen, wie beispielsweise Zuckerrüben, Baumwollpflanzungen, Sojabohnen oder Erdnüssen, als auch in monokotylen Kulturen, insbesondere Getreide, wie beispielsweise Weizen oder Mais, eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, alipha-

0204242

tische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie

natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit N,N-Dimethyl-N'-(3-Chlor-4-methylphenyl)-harnstoff; N,N-Dimethyl-N'-(4-isopropylphenyl)-harnstoff; 4-Amino-6-t-butyl-3-ethylthio-1,2,4-triazin-5(4H)-on, 2,4-Dichlorphenoxyessigsäure; 2,4-Dichlorphenoxypropionsäure; (2-Methyl-4-chlorphenoxy)-essigsäure; (4-Chlor-2- methylphenoxy)-propionsäure; Chloressigsäure-N-(methoxymethyl)-2,6-diethylanilid; 2-Ethyl-6-methyl-N-(1-methyl-2-methoxyethyl)-chloracetanilid; 2-[4-(3,5-Dichlorpyrid-2-yloxy)-phenoxy]-propionsäure-(2-benzyloxyethylester), -(trimethylsilylmethylester) oder -(2,2-diethoxyethylester); Methyl-5-(2,4-dichlorphenoxy)-2-nitrobenzoat; 3,5-Diiod-4-hydroxybenzonitril; 3-Isopropyl-2,1,3-benzothiadiazin-4-on-2,2-dioxid; 2-Chlor-N-<[(4-methoxy-6-methyl-1,3,5-triazin-2-yl)-amino]-carbonyl>-benzolsulfonamid; N-Methyl-2-(1,3-benzthiazol-2-yloxy)-acetanilid; 2-<4-[(3-Chlor-5-(trifluormethyl)-2-pyridinyl)-oxy]-phenoxy>-propansäure bzw. -propansäureethylester; N,N-Diisopropyl-S-(2,3,3-trichlorallyl)-thiocarbamat; 5-(2-Chlor-4-trifluormethylphenoxy)-2-nitrobenzoesäure; 5-(2-Chlor-4-trifluormethylphenoxy)-N-methylsulfonyl-2-nitrobenzamid und 2-Chlor-4-ethylamino-6-isopropylamino-1,3,5-triazin sind von Vorteil.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

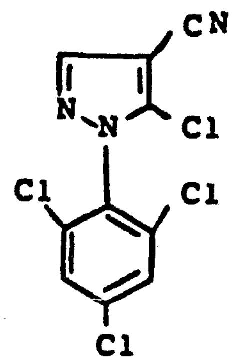

(Verfahren a-β)

Ein Gemisch aus 65,5 g (0,2 Mol) 4-Ethoxycarbonyl-5-hydroxy-1-(2,4,6-trichlorphenyl)-pyrazol-Ammoniumsalz und 150 ml (1,6 Mol) Phosphoroxychlorid wird 15 Stunden im Autoklaven auf 160°C erhitzt. Die erkaltete Reaktionsmischung wird bei Raumtemperatur in eine wässrige Kaliumcarbonatlösung getropft, nach beendeter Zugabe 10 Minuten nachgerührt und abgesaugt. Der feste Rückstand wird mehrere Tage mit Petrolether extrahiert und die Petroletherphase im Vakuum eingedampft.

Man erhält 8 g (13 % der Theorie) an 5-Chlor-4-cyano-1-(2,4,6-trichlorphenyl)-pyrazol vom Schmelzpunkt 83 bis 85°C.

## Herstellung der Ausgangsverbindungen

**Beispiel (III-1)**

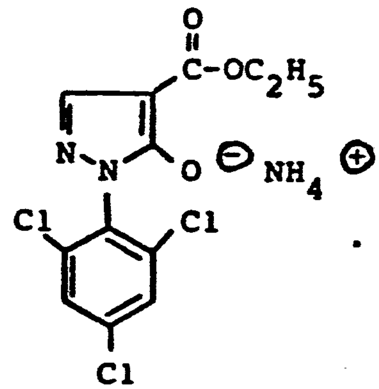

5 g (0,014 Mol) 4-Ethoxycarbonyl-5-hydroxy-1-(2,4,6-trichlorphenyl)-pyrazol in 50 ml Ethanol werden bei Raumtemperatur mit 10 ml 25-prozentiger wässriger Ammoniaklösung versetzt und 24 Stunden unter Rückfluß erhitzt. Die erkaltete Reaktionsmischung wird im Vakuum eingeengt, der Rückstand aus Methanol umkristallisiert und bei 50°C im Vakuum getrocknet.

Man erhält 2,0 g (44 % der Theorie) an 4-Ethoxycarbonyl-5-hydroxy-1-(2,4,6-trichlorphenyl)-pyrazol-Ammoniumsalz vom Schmelzpunkt 152°C.

**Beispiel (IXa-1)**

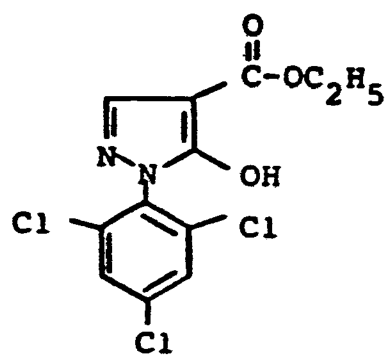

38,5 g (0,094 Mol) N-[2,2-Bis(ethoxycarbonyl)-vinyl]-N'-(2,4,6-trichlorphenyl)-hydrazin werden langsam auf 170°C erhitzt und für 45 Minuten bei 170°C bis 175°C gerührt, wobei gleichzeitig 11 ml Ethanol abdestilliert werden, und der Rückstand langsam fest wird. Der erhaltene Rückstand wird mit Acetonitril verrührt und abgesaugt.

Man erhält 26 g (82,4 % der Theorie) an 4-Ethoxycarbonyl-5-hydroxy-1-(2,4,6-trichlorphenyl)-pyrazol vom Schmelzpunkt 228°C.

Beispiel (XVI-1)

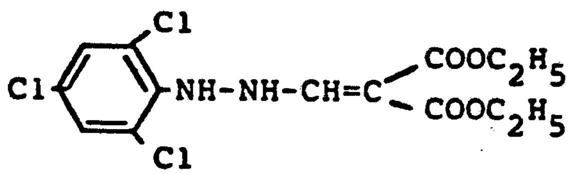

105 g (0,5 Mol) 2,4,6-Trichlorphenylhydrazin und 108 g (0,5 Mol) Ethoxymethylenmalonsäurediethylester werden in 500 ml Ethanol 5 Stunden unter Rückfluß erhitzt, dann auf -10°C abgekühlt. Der Rückstand wird anschließend abfiltriert und getrocknet.

Man erhält N-[2,2-Bis(ethoxycarbonyl)-vinyl]-N'-(2,4,6-trichlorphenyl)-hydrazin vom Schmelzpunkt 92°C.

Beispiel 2

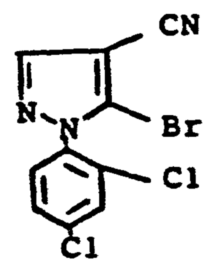

(Verfahren c)

Zu einer Suspension aus 12,7 g (0,05 Mol) 5-Amino-4-cyano-1-(2,4-dichlorphenyl)-pyrazol in 100 ml (4,5 Mol) Bromwasserstoffsäure gibt man bei -5°C bis 0°C 6 g (0,09 Mol) Natriumnitrit in 15 ml Wasser und rührt bis zum Ende der Gasentwicklung, wobei die Temperatur auf 30°C ansteigt. Der feste Rückstand wird abgesaugt, in Wasser aufgeschlämmt, mit Natriumhydrogencarbonat neutralisiert, wieder abgesaugt und getrocknet.

Man erhält 14,5 g (91,5 % der Theorie) an 5-Brom-4-cyano-1-(2,4-dichlorphenyl)-pyrazol von Schmelzpunkt 84°C (Zers.).

In entsprechender Weise und gemäß den allgemeinen Herstellungsangaben erhält man die folgenden 1-Aryl-4-cyano-5-halogenpyrazole der allgemeinen Formel (I):

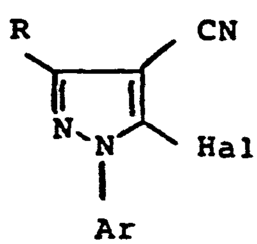

Tabelle 2

| Bsp. Nr. | R | Hal | Ar | Schmelz-punkt/°C |
|---|---|---|---|---|
| 3 | H | Cl | 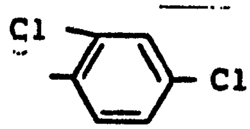 | 81 |
| 4 | H | Cl | 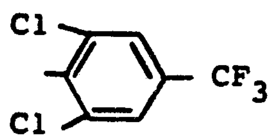 | 68-72 |
| 5 | H | Br | 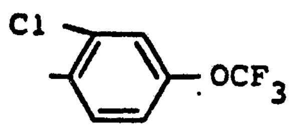 | 83 |
| 6 | H | Br | 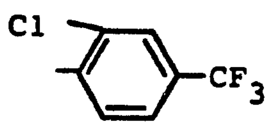 | 127 |
| 7 | H | Br | 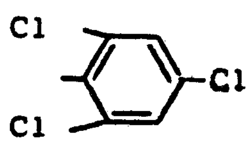 | 102 |
| 8 | H | Br | 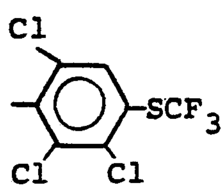 | 95 |
| 9 | H | Br | 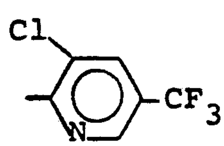 | 102 |

0204242

Tabelle 2 (Fortsetzung)

| Bsp. Nr. | R | Hal | Ar | Schmelzpunkt/°C |
|---|---|---|---|---|
| 10 | H | Br | 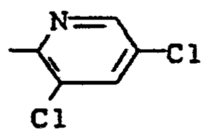 | 142 |
| 11 | H | Cl | 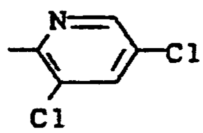 | 126 |
| 12 | H | Br | 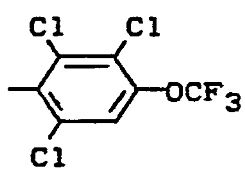 | 81 |
| 13 | H | Br | 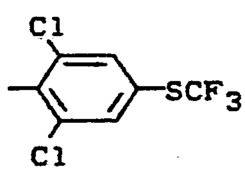 | 137 |
| 14 | H | Br | 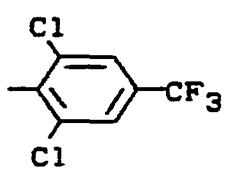 | 92 |

Anwendungsbeispiele

In den folgenden Anwendungsbeispielen wurde die nachstehend aufgeführte Verbindung als Vergleichssubstanz eingesetzt:

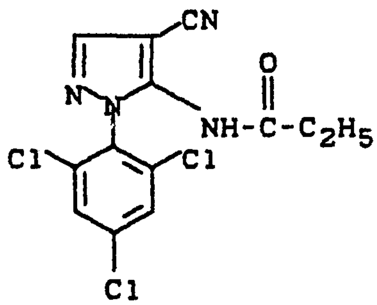

(A)

(bekannt aus DE-OS 3 226 513).

**Beispiel A**

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Nutzpflanzenselektivität, insbesondere in Zuckerrüben und Mais, gegenüber der Vergleichssubstanz (A) zeigt in diesem Test z.B. die Verbindung gemäß dem Herstellungsbeispiel: 6.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Nutzpflanzenselektivität, insbesondere in Soja und Weizen, gegenüber der Vergleichssubstanz (A) zeigen in diesem Test z.B. die Verbindungen gemäß den Herstellungsbeispielen: 6, 9 und 11.

## Patentansprüche

1. 1-Aryl-4-cyano-5-halogenpyrazole der Formel (I),

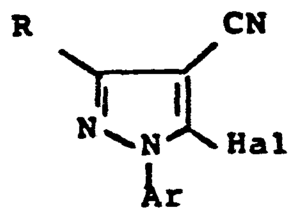

(I)

in welcher

R für Wasserstoff oder Alkyl steht

Hal für Halogen steht und

Ar für substituiertes Phenyl oder für mehrfach substituiertes Pyridyl steht.

2. 1-Aryl-4-cyano-5-halogenpyrazole der Formel (I),

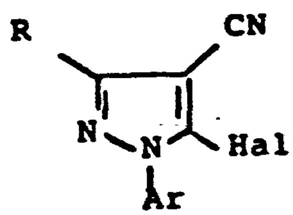

(I)

in welcher

R für Wasserstoff oder für geradkettiges oder verzweigtes Alkyl mit 1 bis 4 Kohlenstoffatomen steht,

Hal für Halogen steht und

Ar für einfach oder mehrfach, gleich oder verschieden substituiertes Phenyl oder für jeweils mehrfach, gleich oder verschieden substituiertes 2-Pyridyl, 3-Pyridyl oder 4-Pyridyl steht, wobei als Substituenten jeweils in Frage kommen:

Cyano, Nitro, Halogen, jeweils geradkettiges oder verzweigtes Alkyl, Alkoxy oder Alkoxycarbonyl mit jeweils bis zu 4 Kohlenstoffatomen, jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder der Rest $-S(O)_n-R^1$, wobei

$R^1$ für Amino, für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

n für eine Zahl 0, 1 oder 2 steht.

3. Verfahren zur Herstellung von 1-Aryl-4-cyano-5-halogenpyrazolen der Formel (I),

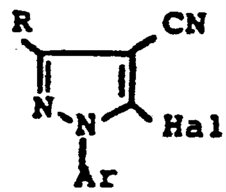

(I)

in welcher

R für Wasserstoff oder Alkyl steht,
Hal für Halogen steht und
Ar für substituiertes Phenyl oder für mehrfach substituiertes Pyridyl steht,

dadurch gekennzeichnet, daß man

(a-α) Pyrazolinone der Formel (II),

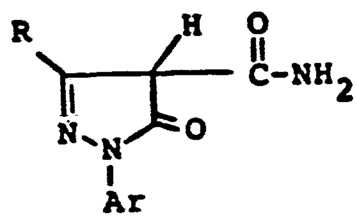

(II)

in welcher

R und Ar die oben angegebene Bedeutung haben,

oder

(a-β) 5-Hydroxypyrazol-Ammoniumsalze der Formel (III)

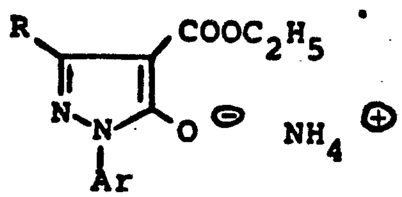

(III),

in welcher

R und Ar die oben angegebene Bedeutung haben,

mit Phosphoroxyhalogeniden der Formel (IV),

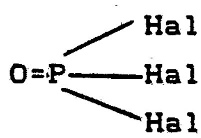

(IV)

in welcher

Hal die oben angegebene Bedeutung hat,

umsetzt,

oder

(b) 4-Hydroximinomethyl-pyrazole der Formel (V),

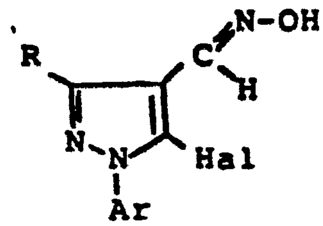

(V)

in welcher

R, Hal und Ar die oben angegebene Bedeutung haben,

mit Acetanhydrid gegebenenfalls in Gegenwart eines Verdünnungsmittels umsetzt,

oder

(c) 5-Amino-4-cyano-pyrazole der Formel (VI),

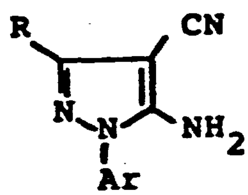

(VI)

in welcher

R und Ar die oben angegebene Bedeutung haben,

mit Natriumnitrit in Gegenwart einer Halogenwasserstoffsäure der Formel (VII),

H-Hal (VII)

in welcher

Hal die oben angegebene Bedeutung hat,

gegebenenfalls in Gegenwart eines Verdünnungsmittels umsetzt,

oder

(d) die nach Verfahren (a), (b) oder (c) erhältlichen 1-Aryl-4-cyano-5-halogenpyrazole der Formel (I),

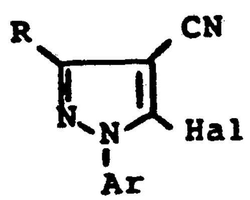

(I)

in welcher

R, Hal und Ar die oben angegebenen Bedeutungen haben,

mit Halogeniden der Formel (VIII),

$$M^{\ominus} \quad (Hal^{\ominus})^1 \qquad \text{(VIII)}$$

in welcher

$M^{\oplus}$ für ein Äquivalent eines Metallkations oder für ein gegebenenfalls substituiertes Ammoniumion steht und

$(Hal^{\ominus})^1$ für ein Halogenanion steht, aber verschieden von Hal ist,

gegebenenfalls in Gegenwart eines Verdünnungsmittels und gegebenenfalls in Gegenwart eines Phasentransferkatalysators umsetzt.

4. Herbizide Mittel, gekennzeichnet durch einen Gehalt an mindestens einem 1-Aryl-4-cyano-5-halogenpyrazol der Formel (I) gemäß Anspruch 1 und 3.

5. Verfahren zur Bekämpfung von Unkräutern, dadurch gekennzeichnet, daß man ein 1-Aryl-4-cyano-5-halogenpyrazol der Formel (I) gemäß Anspruch 1 und 3 auf Unkräuter und/oder ihren Lebensraum einwirken läßt.

0204242

6. Verwendung von 1-Aryl-4-cyano-5-halogenpyrazolen der Formel (I) gemäß Anspruch 1 und 3 zur Bekämpfung von unerwünschtem Pflanzenwuchs.

7. Verfahren zur Herstellung von herbiziden Mitteln, dadurch gekennzeichnet, daß man 1-Aryl-4-cyano-5-halogenpyrazole der Formel (I) gemäß Anspruch 1 und 3 mit Streckmitteln und/oder oberflächenfaktiven Mitteln vermischt.

8. Pyrazolinone der Formel (II),

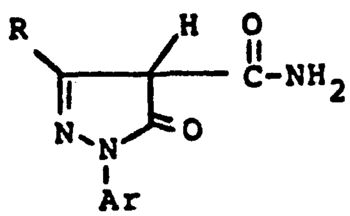

(II),

in welcher

R für Wasserstoff oder Alkyl und

Ar für substituiertes Phenyl oder für mehrfach substituiertes Pyridyl steht.

9. 5-Hydroxypyrazol-Ammoniumsalze der Formel (III),

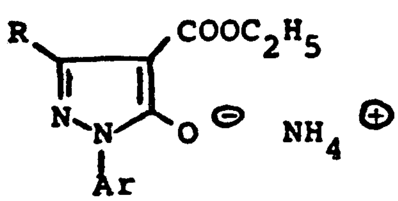

(III)

in welcher

R für Wasserstoff oder Alkyl steht und
Ar für substituiertes Phenyl oder für mehrfach substituiertes Pyridyl steht.

10. 4-Hydroximinomethyl-pyrazole der Formel (V),

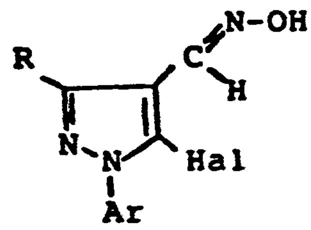

(V)

in welcher

R für Wasserstoff oder Alkyl steht und
Ar für substituiertes Phenyl oder für mehrfach substituiertes Pyridyl steht und
Hal für Halogen steht.